# EUROPEAN PATENT SPECIFICATION

(11) **EP 1 086 980 B1**
(45) Date of publication and mention of the grant of the patent: **12.05.2004**
(21) Application number: 00119339.0
(22) Date of filing: 07.09.2000
(51) Int. Cl.: C08J 7/04, C08F 2/38, A41D 19/015, A61B 19/04

(54) **Use of polymer coating for rubber articles**
Verwendung einer Polymerbeschichtung für Gummigegenstände
Utilisation d'un revêtement polymère pour articles en caoutchouc

(30) Priority: 21.09.1999 US 400488
(43) Date of publication of application: 28.03.2001
(73) Proprietor: National Starch and Chemical Investment Holding Corporation, Wilmington, Delaware 19803-7663 (US)
(72) Inventor: Petrash, Stanislaw, North Brunswick, New Jersey 08902 (US); Xiao, Chaodong, East Hanover, New Jersey 07936 (US); Mukherjee, Apala, Millburn, New Jersey 07041 (US); Thomaides, John S., Berkeley Heights, New Jersey 07922 (US)
(74) Representative: Held, Stephan, Dr.rer.nat., Dipl.-Chem.

(56) References cited:
- EP-A- 0 448 224
- EP-A- 0 594 410
- WO-A-96/37520
- GB-A- 730 921

## Description

The present invention relates to the use of a polymeric coating for rubber articles.

Conventional latex medical gloves are difficult to don without a lubricant. Generally, latex gloves are manufactured with a powdered coating, such as com starch, over the inner surface of the glove so that the gloves can be more easily put on. The powder tends to absorb proteins naturally found in the latex and the powder is easily dislodged during donning and use, contaminating the surrounding environment and causing allergies and other negative effects. Further, the protein/powder complex serves as a food source for bacteria, allowing them to proliferate.

Glove manufacturers have tried to find alternatives to using starch powder to coat gloves. Some latex glove manufacturers use a chlorination process to provide the slippage necessary to facilitate donning of the gloves. In this case, calcium carbonate is used as a mold release agent and washed away prior to chlorination. Although this reduces the tack and friction of the latex, it also makes the rubber less pliant and reduces the shelf life of the glove.

Other manufacturers have looked to alternative coatings. To be an effective substitute for starch, the inner surface coating must not only reduce friction between the latex and the hand to allow convenient donning, but also must allow the latex to stretch without coating delamination. Some glove manufacturers also use a coating on the outside surface of the glove to facilitate release of the latex from the former. Further, the coating should be deliverable from an aqueous solution, be resistant to washing, and meet any relevant regulatory requirements.

Several other types of coatings have been developed, primarily based on polyurethanes: US Patent No. 5,088,125 discloses gloves modified by an ionic polyurethane; US Patent No. 5,272,771 discloses gloves modified by an ionic polyurethane containing fully reacted isocyanate groups; and US 5,534,350 discloses gloves in which the outer glove coating contains a polyurethane dispersion and the inside glove coating contains a polyurethane containing a silicone emulsion.

Other coatings which have been developed include emulsion copolymers, particularly core shell, containing low surface energy monomers and hard monomers as disclosed in US Patent Nos. 5,691,069 and 5,700,585; or containing two monomers selected from styrene, methyl or butyl acrylates, methacrylic or acrylic acid and a silicone oligomer, with glass transition temperatures of less than 0°C and from 0 to 100°C respectively as disclosed in US Patent No. 5,712,346. These sequential emulsion polymerizations lead to substantially linear copolymers.

Surprisingly, it has now been discovered that star-branched polymers, also known as radial polymers, provide both an excellent coating to latex gloves and other rubber latex articles.

The present invention is directed to the use of star polymers as coatings for rubber latex articles, particularly for latex gloves. Star or radial polymers, as used herein, is intended to describe polymers that have three or more polymeric arms emanating from a central core. These polymers can be prepared by various polymerization procedures such as anionic, cationic, and free radical mechanisms. The star polymers are usually formed by using either multifunctional initiators, multifunctional chain transfer agents, or multifunctional coupling agents. The star polymers have unique properties including: low viscosities in solution due to their compact structure and high melt viscosities due to extensive entanglements relative to their linear analogs.

The arms of the star polymers may be of several types: random or block copolymers or homopolymer structures. Arms within a single star structure may have the same or different composition. Further, the arms may or may not contain crosslinkable functionality or other performance improving agents.

When used as a coating for rubber articles, the star polymers provide an inner surface coating that reduces friction between the latex and the hand to allow convenient donning. Star polymers can also be used to facilitate the release of the latex from the former. They are deliverable from aqueous solution and are resistant to washing.

### BRIEF DESCRIPTION OF THE DRAWINGS

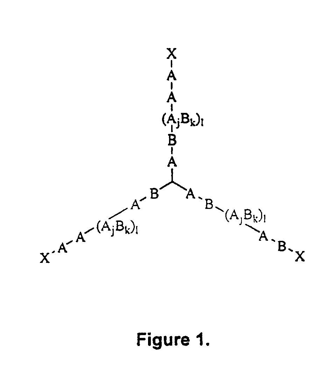

Figure 1 depicts a 3-arm (AB)₃ random star copolymer. j, k, and l are each independent random integers, including zero.

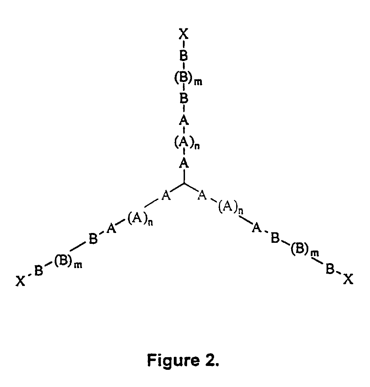

Figure 2 depicts a 3-arm (AB)₃ block star copolymer. m and n are each independent random integers of at least about 8.

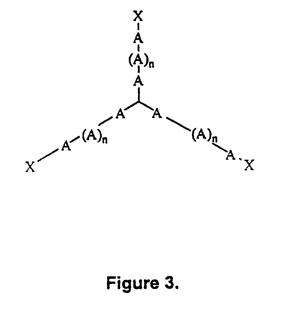

Figure 3 depicts a 3-arm star homopolymer. n is a random integer of at least about 8.

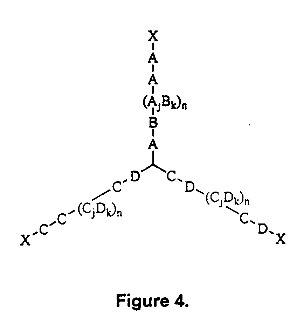

Figure 4 depicts a 3-arm AB(CD)₂ random, heteroarm star copolymer. j, k, and n are each independent random integers, including zero.

The present invention is directed to the use of star polymers as coatings for rubber articles, particularly for latex gloves.

The star polymers of the present invention comprise a polyvalent mercaptan core and three or more polymeric arms which extend radially from the core. The arms comprise homopolymers, random copolymers, or block copolymers. Further, arms within a single star structure may have the same or different composition.

As used herein, homopolymer is intended to mean an arm which consists of multiple units of essentially only one monomer; a random copolymer arm is intended to mean an arm which consists of at least two different monomers in a random distribution; and a block copolymer arm is intended to mean an arm which consists of a linear arrangement of blocks of varying monomer composition. Also, as used herein, heteroarm is intended to mean a star copolymer in which at least one arm of the star copolymer has a composition that is significantly different from that of the other arms.

The polyvalent mercaptan core of the present invention comprises three or more thiol groups. The thiol groups can be either all the same or all different or variations therein. It is at the thiol groups that the monomers will react to create the polymeric arms of the star polymer.

Specifically, the polyvalent mercaptan core comprises a central component, derived from a multifunctional alcohol which has been substituted with thiol derivatives. The multifunctional alcohol can have any number of functional hydroxy units, particularly three to eight functional units. To prepare the core of the present invention, each of the OH functional units will be substituted with thiol units. Accordingly, in a particularly suitable embodiment the core compositions range from a tri-functional alcohol substituted with three identical thiol groups, up to an octa-functional alcohol substituted with eight different thiol groups.

In one embodiment of the present invention, the polyvalent mercaptan core is of the general formula:

X-(Y₁-SH)ₐ(Y₂-SH)_{b}(Y₃-SH)_{c}(Y₄-SH)_{d}(Y₅-SH)ₑ(Y₆-SH)_{f}(Y₇-SH)_{g}(Y₈-SH)ₕ

or one of the following specific embodiments:

(HS-Y₁)ₐ-X (I)

(HS-Y₁)ₐ-X-(Y₂-SH)_{b} (II)

X-(Y₁-SH)ₐ(Y₂-SH)_{b}(Y₃-SH)_{c} (III)

X-(Y₁-SH)ₐ(Y₂-SH)_{b}(Y₃-SH)_{c}(Y₄-SH)_{d} (IV)

X-(Y₁-SH)ₐ(Y₂-SH)_{b}(Y₃-SH)_{c}(Y₄-SH)_{d}(Y₅-SH)ₑ (V)

X-(Y₁-SH)ₐ(Y₂-SH)_{b}(Y₃-SH)_{c}(Y₄-SH)_{d}(Y₅-SH)ₑ(Y₆-SH)_{f} (VI)

X-(Y₁-SH)ₐ(Y₂-SH)_{b}(Y₃-SH)_{c}(Y₄-SH)_{d}(Y₅-SH)ₑ(Y₆-SH)_{f}(Y₇-SH)_{g} (VII)

or

X-(Y₁-SH)ₐ(Y₂-SH)_{b}(Y₃-SH)_{c}(Y₄-SH)_{d}(Y₅-SH)ₑ(Y₆-SH)_{f}(Y₇-SH)_{g}(Y₈-SH)ₕ (VIII)

where X is derived from an organic radical having a valence of 3 to 8. In particular, X is derived from a tri- to octa- multi-functional alcohol such as glycerol, sorbitol, trimethylolpropane, pentaerythritol, dipentaerythritol, tripentaerythritol, and inositol.

Variables Y_{1:} Y₂, Y_{3:} Y₄, Y₅, Y₆, Y₇ and Y₈ are the same or different and each comprises C₂₋₁₀ alkanoic acids, particularly C₂₋₆ alkanoic acids. Variables a, b, c, d, e, f, g and h are integers from 0 to 8 provided that a+b+c+d+e+f+g+h ≤ 8. If the core is a core of differential reactivity, or heterocore, a and b are integers from 1 to 8, and c, d, e, f, g, h are integers from 0 to 8 provided that a+b+c+d+e+f+g+h ≤ 8. If the core is a homocore, b = c = d = e = f = g = h = 0.

Each of the above identified (Y-SH) units are derived from, for example, 2-mercaptoacetic acid, 2-mercaptopropionic acid, 3-mercaptopropionic acid, 4-mercaptobutyric acid, 5-mercaptopentanoic acid, or 6-mercaptohexanoic acid, particularly 2-mercaptopropionic acid and 3-mercaptopropionic acid.

Examples of cores of differential reactivity include pentaerythritol bis(3-mercaptopropionate) bis(2-mercaptopropionate); trimethylolpropane bis(3-mercaptopropionate)(2-mercaptopropionate); pentaerythritol tris(3-mercaptopropionate)(2-mercaptopropionate); pentaerythritol tris(2-mercaptopropionate) (3-mercaptopropionate) and trimethylolpropane bis(2-mercaptopropionate)(3-mercaptopropionate).

Cores of non-differential reactivity, or homocores, include pentaerythritol tetrakis(3-mercaptopropionate), pentaerythritol tetrakis(2-mercaptoacetate); pentaerythritol tetrakis(2-mercaptopropionate), trimethylolpropane trithiopropionate, pentaerythritol tetrakis(thioglycolate) and dipentaerythritol hexakis thioglycolate, particularly pentaerythritol tetrakis(3-mercaptopropionate); pentaerythritol tetrakis(2-mercaptoacetate); and pentaerythritol tetrakis(2-mercaptopropionate)..

In general, the polyvalent mercaptan core is prepared by reacting a multi-functional alcohol with the appropriate amount of mercapto acid to prepare the polyvalent mercaptan core. For example, if the multifunctional alcohol is a tri-alcohol, three equivalents of mercapto acid are added to give three (HS-Y) units. The three equivalents of mercapto acid can be made up of any combination of the suitable mercapto acids. For example, one equivalent of 2-mercaptopropionic acid (a secondary thiol-containing acid) and two equivalents of 3-mercaptopropionic acid (a primary thiol-containing acid) will provide a core of differential reactivity. If the alcohol is a trialcohol and three equivalents of 2-mercaptopropionic acid are used, a homocore within the scope of the present invention is also obtained.

In a specific example, pentaerythritol can be used as the multifunctional alcohol, X, used to prepare the core. To pentaerythritol is added two mole equivalents each of a primary thiol, 3-mercaptopropionic acid, and a secondary thiol, 2-mercaptopropionic acid. The result will be a mixture of five compounds corresponding to molecules containing ratios of primary/secondary SH groups of 0/4, 1/3, 2/2, 3/1, and 4/0. Those cores with ratios of 1/3, 2/2 and 3/1 have differential reactivity and are within the scope of the present invention. The cores with 0/4 and 4/0 are homocores. The product mixture, though a statistical mixture, has cores with an average of two primary thiol groups and two secondary thiol groups per core as shown by the following reaction:

The reaction is shown below:

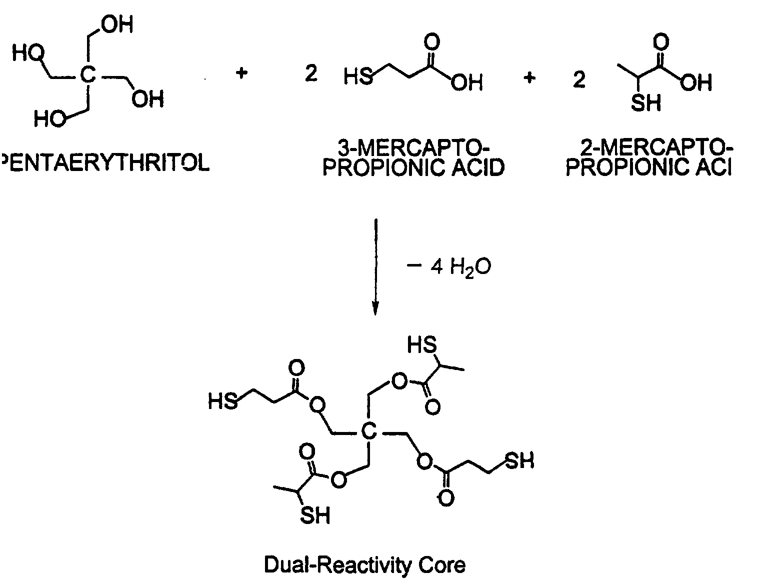

With dipentaerythritol, seven possible compounds can be obtained corresponding to 0,1,2,3,4,5 and 6 primary SH groups per molecule. These differential thiols will be utilized to provide enhanced selectivity to generate heteroarm stars.

In an alternative embodiment, four mole equivalents of thiol can be added to pentaerythritol:

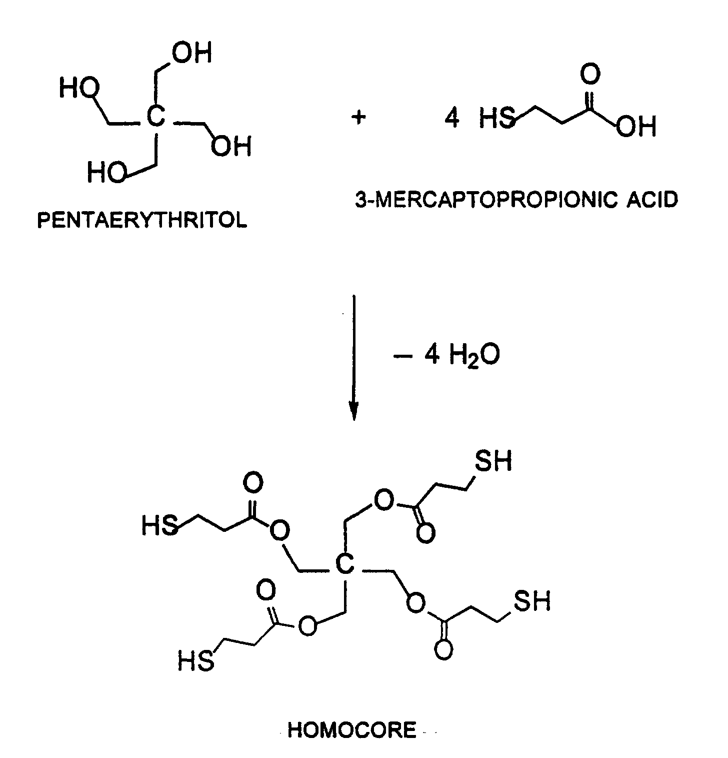

The star polymers of the present invention are formed using the mercaptan core as a chain transfer agent in polymerization processes which include bulk, solution, emulsion, and suspension polymerization. A particularly suitable process is the solution polymerization process employing a free radical initiator. The polymerization reaction is typically conducted at temperatures in the range of 10 to 120°C, particularly 70 to 100°C.

The present invention contemplates that the resulting polymer may comprise arms that are all the same, or all the same after the S atom but with different Y connecting groups.

The preparation of the star polymers of the present invention may be by the sequential or non-sequential addition of monomers to the mercaptan core. In this embodiment, the core may be a core of differential reactivity, or a homocore. The polymerization proceeds by chain transfer to the mercapto group from the growing polymer chains or initiator radicals. As a result, a structure in which polymer arms which radiate from a central core are produced. It is recognized that a small amount of linear copolymers may also be produced.

In general, the orders of reactivity of thiol groups are: SH groups attached to aromatic rings (i.e., thiophenols) are more reactive than SH groups attached to primary aliphatic carbon atoms which are more reactive than SH groups attached to secondary aliphatic carbon atoms, i.e., ArSH>RCH₂SH> RR'CHSH.

In the above embodiment, the monomer mixtures can be added by any method familiar to the skilled artisan including dropwise or by slug dose.

Monomers that may be used to prepare the polymeric arms of the star polymers of the present invention are typically vinyl or ethylenically unsaturated monomers and optionally may contain crosslinkable functionality. The term "crosslinkable functionality" herein refers to functional groups such as hydroxyl, carboxyl, silanes, siloxanes, isocyanates, epoxides, acetoacetonates, and combinations thereof that self-react, react with other polymer-bound functionality, or react with multifunctional cross-linking agents to form stable bonds. The star polymer may include essentially any ethylenically unsaturated monomer or mixtures of ethylenically unsaturated monomers copolymerizable with the other components.

Examples of vinyl unsaturated monomers include those selected from the group consisting of acrylic and methacrylic acids, acrylamide and methacrylamide, acrylonitrile and methacrylonitrile, alkoxyalkyl acrylamides and methacrylamides, e.g., butoxymethyl acrylamide and methoxymethyl methacrylamide, hydroxyalkyl acrylamides e.g., N-methylol acrylamide, and olefins, e.g. 1-butene and 1,3-butadiene. Particularly suitable are the esters and amides of acrylic and methacrylic acids with alcohols, phenols and amines; the vinyl aromatic compounds, e.g., styrene, alpha-methylstyrene and substituted derivatives thereof such as the halogenated derivatives thereof and vinyl toluene; the vinyl esters, vinyl amides, e.g., vinyl acetate and 1-vinyl-2-pyrrolidinone; and ethylenically unsaturated nitriles, e.g. acrylonitrile, and combinations thereof.

Monomers may be selected from hydroxyalkyl esters of ethylenically unsaturated carboxylic acids, ethylenically unsaturated carboxylic acids, ethylenically unsaturated epoxides, ethylenically unsaturated isocyanates and combinations thereof.

Other unsaturated monomers include hydroxypropyl (meth)acrylate, glycidyl (meth)acrylate, methoxyethyl (meth)acrylate, ethoxyethyl (meth)acrylate, ethoxyethoxyethyl (meth)acrylate, cyanoethyl acrylate and the like; vinyl ethers which are represented by methyl vinyl ether, ethyl vinyl ether, isobutyl vinyl ether, and the like; fumaric acid, monoalkyl fumarates, dialkyl fumarates; maleic acid, monoalkyl maleates, dialkyl maleates; itaconic acid, monoalkyl itaconates, dialkyl itaconates; half esters of succinic anhydride or phthalic anhydride with hydroxyethyl (meth)acrylate; (meth)acrylonitrile, butadiene, isoprene, vinyl halides, e.g. vinyl chloride and vinylidene chloride, vinyl ketones, vinyl pyridine, vinyl carbazole, and the like. These compounds may be used either alone or in combinations with each other.

The present invention also contemplates the use of multifunctional monomers such as ethylene glycol di(meth)acrylate, trimethylolpropane tri(meth)acrylate, diethylene glycol di(meth)acrylate trisacrylate, divinyl benzene, triallyl cyanurate, allyl acrylate, diallyl phthalate, diallyl sucrose and allyl(meth)acrylate.

Particularly suitable vinyl monomers include those that have chain transfer constants with thiols that are close to one. Such monomers include those of the formula CH₂=C(R)COOR' where R is H or methyl and R' is H or C₁. ₂₂ alkyl, C₅₋₁₀ cycloalkyl or C₆₋₁₀ aryl wherein the alkyl, cycloalkyl or aryl is optionally substituted with halogen or hydroxyl. Examples of such monomers include acrylic and methacrylic acid and esters of acrylic acid and methacrylic add such as methyl acrylate, ethyl acrylate, n-butyl acrylate, 2-hydroxyethyl acrylate, hydroxypropyl acrylate, isobomyl acrylate, lauryl acrylate, stearyl acrylate, methyl methacrylate, ethyl methacrylate, n-propyl methacrylate, isopropyl methacrylate, n-butyl methacrylate, isobutyl methacrylate, cyclohexyl methacrylate, isobomyl methacrylate, benzyl methacrylate, phenyl methacrylate, lauryl methacrylate, stearyl methacrylate, hydroxyethyl methacrylate and hydroxypropyl methacrylate.

Methyl methacrylate, methyl acrylate, acrylic acid, methacrylic acid, n-butyl methacrylate, n-butyl acrylate, and t-octyl acrylamide are the most particularly useful monomers.

The polymeric arms of the star polymer may also optionally include a small amount of an olefinic monomer containing crosslinkable functionality such as alcohols, acids, silanes, siloxanes, isocyanates, and epoxides, particularly silanes and siloxanes. Examples of such monomers include, but are not limited to, hydroxyalkyl esters of ethylenically unsaturated carboxylic acids, ethylenically unsaturated carboxylic acids, ethylenically unsaturated epoxides and ethylenically unsaturated isocyanates. Particularly suitable crosslinkers include vinyltriisopropoxysilane, vinyltrimethoxysilane, vinyltriethoxysilane, vinyl-tris(2-methoxy-ethoxy)silane, and gamma-methacryloxyoropyltrimethoxysilane.

The polymeric arms of the star polymer may also include performance improving agents, such as low surface energy modifiers and monomers which promote adhesion of the coating material to the latex by providing a cationic charge. Low surface energy modifiers, as used herein, is intended to mean a monomer which if homopolymerized would require only a low level of force to release it from the surface to which it is applied, and include fluorocarbons, silicones, and fatty acid esters having vinyl, acrylic and/or methacrylic functionalities, particularly silicone acrylates, silicone methacrylates, C₁₋₁₂ perfluoroalkyl ethyl acrylates, C₁₋₁₂ perfluoroalkyl ethyl methacrylates, [2-(N-ethylperfluoroalkylsulfonamido)ethyl acrylates, and [2-(N-ethylperfluoroalkylsulfonamido)ethyl methacrylates. Such performance improving agents may be added in any amount necessary to obtain the desired characteristics of the polymer, particularly from about 0.5 to about 20%, particularly from about 1 to 15%, by weight of the polymer.

The combination of monomers used to make up the star polymer and the exact relative amounts of each will be such as to provide a polymeric coating material appropriate for the end use application. For use as a coating for latex gloves, the polymer should have a low coefficient of friction, whether wet or dry, to facilitate donning of the gloves; and a high elongation-to-break, particularly at least about 300%, more particularly at least about 500%, most particularly at least about 700%, when self bonded to a latex surface so as to allow the glove to stretch without peeling of the polymeric coating. This is typically accomplished by selecting at least one monomer which forms a soft (low T_{g}) block which is compatible with latex and has high elongation and at least one additional monomer which forms a hard (high T_{g}) block to provide good slippage. T_{g}, as used herein, is intended to denote glass transition temperature.

A particularly suitable structure is a heteroarm star polymer in which at least one arm is rich in monomers which provide a soft block and at least one arm rich in monomers which provide a hard block. More particularly, the star polymer has at least one arm with a T_{g} of at least about 20°C and at least one arm with a T_{g} of at less than about 20°C. Most particularly the star polymer has at least one arm with a T_{g} of at least about 40°C and at least one arm with a T_{g} of at no more than about 0°C.

It is also desirable that the polymer be deliverable from an aqueous solution, resistant to washing, and meet regulatory requirements. Thus, a particularly suitable star copolymer will contain at least one hydrophilic monomer and at least one hydrophobic monomer. In order to be deliverable from an aqueous solution, the material must be sufficiently rich in hydrophilic monomer. Suitable hydrophilic monomers include those monomers that are ionic, e.g. anionic, cationic, or zwitterionic, or have sufficient nonionic polar functionality, e.g. hydroxyl or amido groups to render them hydrophilic.

Anionic monomers typically contain carboxylic, sulfonic, and/or phosphonic acid functionality. Exemplary anionic monomers include, without limitation, (meth)acrylic acid and the alkali, alkaline earth or ammonium salts thereof, crotonic acid and the alkali, alkaline earth or ammonium salts thereof, dicarboxylic acid containing monomers such as maleic, fumaric and itaconic acids including the anhydrides and the alkali, alkaline earth or ammonium salts thereof, the half esters of unsaturated dicarboxylic acids such as methyl hydrogen fumarate, butyl hydrogen fumarate, ethyl hydrogen maleate, and butyl hydrogen maleate and the alkali, alkaline earth or ammonium salts thereof, the allyloxybenzenesulfonate monomers described in U.S. patent No. 4,892,898, methallylsulfonate and the alkali, alkaline earth or ammonium salts thereof, styrene sulfonic acid and the alkali, alkaline earth or ammonium salts thereof, acrylamido alkyl or aryl sulfonates such as 2-acrylamido-2-methyl propane sulfonic acid and the alkali, alkaline earth or ammonium salts thereof, vinyl sulfonic acid and the alkali, alkaline earth or ammonium salts thereof, and vinyl phosphonic acid and the alkali, alkaline earth or ammonium salts thereof.

Cationic monomers typically contain amine or quaternary ammonium functionality. Exemplary cationic monomers include, without limitation, (3-acrylamidopropyl)trimethylammonium chloride, [2-(acryloyloxy)ethyl]-trimethylammonium methyl sulfate, 2-(dimethylamino)ethyl acrylate, [3-(methacryloylamino)propyl]trimethylammonium chloride, [2-(methacryloyloxy) ethyl]trimethylammonium chloride, [2-(methacxyloyloxy)-ethyl]trimethylammonium methyl sulfate, 2-(dimethylamino)ethyl methacrylate, 2-aminoethyl methacrylate hydrochloride, diallyldimethylammonium chloride, vinyl imidazole, and vinyl pyridine and substituted derivatives thereof.

Zwitterionic monomers typically consist of, without limitation, unsaturated carboxyl, sulfoxyl or sulfate-substituted amines, and are commonly referred to as betaines. Exemplary zwitterionic monomers include, without limitation, [3-(methacryloylamino)propyl]dimethyl(3-sulfopropyl)ammonium hydroxide inner salt and [2-(methacryloyloxy)ethyl]dimethyl(3-sulfopropyl)ammonium hydroxide, inner salt hydrate.

Exemplary monomers that have sufficient nonionic polar functionality to render them hydrophilic include, without limitation C₁₋₄ hydroxy alkyl esters of (meth)acrylic acid, e.g. 2-hydroxyethyl acrylate, and hydroxypropyl acrylate, 2-hydroxyethyl methacrylate, hydroxypropyl methacrylate, vinyl formamide, vinyl acetamide, 1-vinyl-2-pyrrolidinone, methacrylamide, acrylamide, C₁₋₄ mono- and dialkyl substituted (meth)acrylamides, e.g. N,N-dimethylacrylamide and N-isopropylacrylamide, acrylonitrile, and methacrylonitrile.

Particularly suitable hydrophilic monomers include methacrylic acid, acrylic acid, 2-acrylamido-2-methyl propane sulfonic acid and the alkali, alkaline earth or ammonium salts thereof, and [3-(methacryloylamino)propyl]-trimethylammonium chloride.

In order to be washable, the material must be sufficiently rich in hydrophobic monomer. Suitable hydrophobic monomers include those of the formula CH₂=C(R)COOR' where R is H or methyl and R' is C₁₋₂₂ alkyl, C₅₋₁₀cycloalkyl or C₆₋₁₀ aryl wherein the alkyl, cycloalkyl or aryl is optionally substituted with halo groups. Examples of such monomers include methyl acrylate, ethyl acrylate, n-butyl acrylate, isobornyl acrylate, 2-ethylhexyl acrylate, lauryl acrylate, stearyl acrylate, methyl methacrylate, ethyl methacrylate, n-propyl methacrylate, isopropyl methacrylate, n-butyl methacrylate, isobutyl methacrylate, cyclohexyl methacrylate, isobomyl methacrylate, benzyl methacrylate, 2-ethylhexyl methacrylate, lauryl methacrylate, and stearyl methacrylate.

Other hydrophobic monomers include vinyl esters, e.g. vinyl acetate, vinyl propionate, vinyl 2-ethylhexanoate, vinyl pivalate, vinyl neononanoate, vinyl neodecanaote, and vinyl stearate, vinyl benzene compounds, e.g. styrene, alpha-methylstyrene and substituted derivatives thereof such as the halogenated derivatives thereof and vinyl toluene; and C₅₋₁₈ mono- and dialkyl (meth)acrylamides, e.g. t-octyl acrylamide.

Particularly suitable hydrophobic monomers include methyl methacrylate, methyl acrylate, n-butyl methacrylate, n-butyl acrylate, 2-ethylhexyl methacrylate, 2-ethylhexyl acrylate, lauryl methacrylate, lauryl acrylate, and t-octyl acrylamide.

In one embodiment of the present invention, the arms of the star polymer are prepared from one or more monomers selected from the group consisting of the alkyl esters of acrylic or methacrylic acids along with a small proportion of one or more hydrophilic monomer selected from the group consisting of methacrylic acid, acrylic acid, 2-acrylamido-2-methyl propane sulfonic acid and the alkali, alkaline earth or ammonium salts thereof, and [3-(methacryloylamino)propyl]trimethylammonium chloride. The star polymer may contain optionally a monomer with crosslinkable functionality. The star polymer may also contain optionally performance improving agents such as low surface energy modifiers.

In another embodiment of the present invention, the arms of the star polymer are prepared from one or more monomers selected from the group consisting of the alkyl esters of acrylic or methacrylic acids along with a small proportion of one or more hydrophilic monomers selected from the group consisting of methacrylic acid, acrylic acid, 2-acrylamido-2-methyl propane sulfonic acid and the alkali, alkaline earth or ammonium salts thereof, and [3-(methacryloylamino)propyl]trimethylammonium chloride and at least one arm of the star polymer has a composition that is substantially different from that of the other arms. The star polymer may contain optionally a monomer with crosslinkable functionality. The star polymer may also contain optionally performance improving agents such as low surface energy modifiers.

When the polymeric arm is prepared from acrylic and methacrylic monomer units, the polymer arm of the resulting polymer comprises from about 10 to 1500 monomer units, particularly from about 20 to 500. When a mixture of monomers is used, the copolymer may be a random or block copolymer of such units.

Free radical initiators suitable for use in the polymerization process of the present invention include, for example: azo-based polymerization initiators such as 2,2'-azobisisobutyronitrile ("AIBN") and 2,2'-azobis(cyclohexanecarbonitrile); peroxide-based polymerization initiators such as benzoyl peroxide; and the like, particularly azo-based initiators. Other suitable initiators include organic peroxides, hydroperoxides, persulfates and azo compounds such as methyl ethyl ketone peroxide, cumene hydroperoxide, potassium persulfate, lauroyl peroxide, 2,5-dimethyl-2,5-di(t-butylperoxy)hexane, diethyl peroxide, dipropyl peroxide, dilauryl peroxide, dioleyl peroxide, distearyl peroxide, di(tertiary butyl) peroxide, di(tertiary amyl) peroxide, tertiary butyl hydroperoxide, tertiary amyl peroxide, acetyl peroxide, propionyl peroxide, lauroyl peroxide, stearoyl peroxide, malonyl peroxide, succinyl peroxide, phthaloyl peroxide, acetyl benzoyl peroxide, propionyl benzoyl peroxide, ascaridole, ammonium persulfate, sodium persulfate, sodium percarbonate, potassium percarbonate, sodium perborate, potassium perborate, sodium perphosphate, potassium perphosphate, tetralin hydroperoxide, tertiary butyl diperphthalate, tertiary butyl perbenzoate, 2,4-dichlorobenzoyl peroxide, urea peroxide, caprylyl peroxide, p-chlorobenzoyl peroxide, 2,2-bis(tertiary butyl peroxy)butane, hydroxyheptyl peroxide and dialkyl esters of peroxydicarbonate.

The reaction typically requires a solvent. The solvent can be selected from the group consisting of organic solvents which are represented by: aromatic hydrocarbons such as toluene and xylene; esters such as ethyl acetate and butyl acetate; cycloaliphatic hydrocarbons such as cyclohexane; aliphatic hydrocarbons such as hexane and pentane; ketones such as acetone, methyl ethyl ketone, and methyl isobutyl ketone; aliphatic esters; alcohols such as isopropanol; and the like. Other suitable solvents include naphthalene, trichlorobenzene, dimethylformamide, and dimethylacetamide. Particularly suitable solvents are alcohols, more particularly isopropanol.

After the radial polymer is formed, the solvent may then be removed and water added to form a stable colloid. If carboxylic acid functionality is used, a base is generally added to neutralize the acid and thus introduce a hydrophilic charge. Suitable bases include alkali and alkali earth metal hydroxides, carbonates or bicarbonates and ammonium hydroxide, particularly sodium hydroxide, potassium hydroxide, calcium hydroxide, ammonium hydroxide, sodium bicarbonate, potassium bicarbonate, sodium carbonate, more particularly sodium hydroxide, and ammonium hydroxide.

The amount of water added is dependent upon the end use application. For example, for coating latex gloves, the polymer typically is present in an amount necessary to provide the desired coating by dipping, particularly at least about 2%, more particularly at least about 5% solids by weight; and particularly no more than about 30%, more particularly no more than about 20 % solids by weight.

The polymeric coating may be used to coat a variety of latex items, including gloves, prophylactics, catheters, balloons, tubing, and sheeting. The polymeric coating may also be used to coat such items made of synthetic rubbers. A particularly suitable end use application is the coating of latex gloves, including surgeons' gloves, physicians' examining gloves, and workers' gloves, more particularly powder-free latex gloves. Such coating may be used on the inside of the glove to provide slippage and promote donning and/or on the outside to facilitate release from the glove form and prevent sticking together during storage.

When used to coat gloves, the polymeric colloid may be applied using standard methods known in the art. For example, one conventional method of making latex gloves is to dip a former or mold in the shape of a hand into a coagulant mixture containing calcium nitrate. After drying, the mold is immersed in a latex emulsion for a time sufficient for the rubber to coagulate and form a coating of the desired thickness. Optionally, the glove then may be water leached to remove rubber impurities. The formed glove is then oven cured and cooled. After cooling, the glove is stripped from the mold and inverted. To use a star polymer coating as a mold release agent, the star polymer may be included in the coagulant mixture. To coat the inside of the glove, the star polymer may be applied immediately before latex curing.

The star polymer used as a mold release agent and the star polymer used as an inner glove coating may be the same of different. Further, each star polymer may comprise a single star polymer or a mixture of at least two star polymers.

An adhesion promoter may be used, and for some polymers may be necessary, to add charge and increase the amount of polymer picked up by the latex. Such adhesion promoter is typically a water soluble salt such as sodium, calcium, zinc, aluminum salts, particularly sodium chloride and calcium nitrate. The salt is typically provided in a concentration of up to about 40%, particularly from about 20 to about 40% by weight of coating suspension. The adhesion promoter is generally applied after leaching.

The polymer coats and adheres to the glove or other latex article undergoing formation, but may also be partially absorbed therein. In the alternative, the polymer may be deposited on a preformed latex article.

The latex article, i.e. glove, may be formed so that the star polymer coats the inside and/or the outside surface of the article. The polymer provides the desired glove properties without the need for chlorination or other coatings, including powders. However, if only one surface is coated, chlorination or another coating may be used to provide the desired properties on the non-star polymer coated surface.

A glove may be made by a method comprising
a) dipping a former into a coagulant that comprises a mold release agent, removing the former from the coagulant and drying it to form a mold-releasing layer on the former; b) dipping the former into rubber latex and drying to form a partially-cured rubber deposit on top of mold-release layer; c) dipping the deposit of rubber into a dispersion of at least one star polymer, and drying it to form a lubricating layer on the rubber deposit; d) vulcanising the deposit of rubber with the mold-releasing and lubricating layers in an oven at about 100°C until the rubber is vulcanised to the desired degree and the layers are bonded to the rubber; and e) cooling and then removing a finished glove from the said ceramic former. The mold release agent of the coagulant is preferably at least a star polymer. In a preferred embodiment the method of making a glove further comprises after step (b) dipping the partially cured rubber deposit into water for sufficient time to remove at least some soluble proteins and other contaminants from the partially cured rubber deposit to form a leached partially cured rubber deposit.

The following examples are presented to further illustrate and explain the present invention and should not be taken as limiting in any regard.

### EXAMPLES

### Example 1 - Preparation of a Heteroarm Star Copolymer

A mixture of 45 parts methyl methacrylate, 10 parts methacrylic acid, 6.5 parts pentaerythritol tetrakis (3-mercaptopropionate) and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts 2,2'-azobisisobutyronitrile was added to the reaction vessel and it was heated at reflux for 45 minutes. 45 Parts butyl acrylate was added to the reaction vessel and heating under reflux was continued for 2 hours. The reaction was cooled and 100 parts of 1.25% ammonium hydroxide was added and stirred for 30 minutes. The isopropyl alcohol was then removed and water was added to make a stable colloid. The final polymer was neutralized to a pH of 7.0. The polymer was characterized through molecular weight (GPC), surface tension, viscosity and % solids.

### Example 2 - Preparation of Heteroarm Star Copolymer with Silane Crosslinker

A mixture of 40 parts methyl methacrylate, 5 parts vinyltriisoproxysilane, 10 parts methacrylic acid, 6.5 parts pentaerythritol tetrakis (3-mercaptopropionate) and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts 2,2'-azobisisobutyronitrile was added to the reaction vessel and it was heated at reflux for 45 minutes. 45 Parts butyl acrylate was added to the reaction vessel and heating under reflux was continued for 2 hours. The reaction was cooled and 100 parts of 1.25% ammonium hydroxide was added and stirred for 30 minutes. The isopropyl alcohol was then removed and water was added to make a stable colloid. The final polymer was neutralized to a pH of 7.0. The polymer was characterized through molecular weight (GPC), surface tension, viscosity and % solids.

### Example 3 - Preparation of Heteroarm Star Copolymer with Silane Crosslinker

A mixture of 40 parts butyl methacrylate, 5 parts vinyltriisoproxysilane, 6.5 parts pentaerythritol tetrakis (3-mercaptopropionate) and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts 2,2'-azobisisobutyronitrile was added to the reaction vessel and it was heated at reflux for 45 minutes. 10 Parts butyl acrylate, 5 parts vinyltriisopropoxysilane and 40 parts acrylic acid were added to the reaction vessel and heating under reflux was continued for 2 hours. The reaction was cooled and 100 parts of 1.25% ammonium hydroxide was added and stirred for 30 minutes. The isopropyl alcohol was then removed and water was added to make a stable colloid. The final polymer was neutralized to a pH of 9.5. The polymer was characterized through molecular weight (GPC), surface tension, viscosity and % solids.

### Example 4 - Preparation of Heteroarm Star Copolymer with MAPTAC

A mixture of 45 parts methyl methacrylate, 6.5 parts pentaerythritol tetrakis (3-mercaptopropionate), 10 parts of 50% MAPTAC and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts 2,2'-azobisisobutyronitrile was added to the reaction vessel and it was heated at reflux for 45 minutes. 45 Parts butyl acrylate was added to the reaction vessel and heating under reflux was continued for 2 hours. The reaction was cooled and 100 parts water was added and stirred for 30 minutes. The isopropyl alcohol was then removed and water was added to make a stable colloid. The final polymer was neutralized to a pH of 7.0. The polymer was characterized through molecular weight (GPC), surface tension, viscosity and % solids.

### Example 5 - Preparation of Heteroarm Star Copolymer with Silicone

A mixture of 35 parts methyl methacrylate, 6.5 parts pentaerythritol tetrakis (3-mercaptopropionate), 10 parts methacrylic acid, 10 parts TEGO®RAD 2700 (a silicone acrylate commercially available from Goldschmidt) and 100 parts isopropyl alcohol is placed in a four-necked 1-liter round-bottomed flask equipped with a nitrogen inlet, condenser and thermometer. The reactor is heated to 75°C while stirring under atmospheric nitrogen. 0.5 Parts 2,2'-azobisisobutyronitrile is added to the reaction vessel and it is heated under reflux for 45 minutes. 45 Parts butyl acrylate is added to the reaction vessel and heating under reflux is continued for 2 hours. The reaction is cooled and 100 parts of 1.25% ammonium hydroxide is added and stirred for 30 minutes. The isopropyl alcohol is then removed and water is added to make a stable colloid. The final polymer is neutralized to a pH of 7.0. The polymer is characterized through molecular weight (GPC), surface tension, viscosity and % solids.

### Example 6 - Preparation of Random Star Copolymer

A mixture of 45 parts methyl methacrylate, 10 parts methacrylic acid, 45 parts butyl acrylate, 6.5 parts pentaerythritol tetrakis (3-mercaptopropionate) and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts 2,2'-azobisisobutyronitrile was added to the reaction vessel and it was heated under reflux for 2 hours. The reaction was coded and 100 parts of 1.25% ammonium hydroxide was added and stirred for 30 minutes. The isopropyl alcohol was then removed and water was added to make a stable colloid. The final polymer was neutralized to a pH of 7.0.

### Example 7 - Preparation of Linear Copolymer

A mixture of 45 parts methyl methacrylate, 10 parts methacrylic acid, 45 parts butyl acrylate, 26 parts 1-dodecanethiol and 100 parts isopropyl alcohol was placed in a four-necked 1-liter round-bottomed flask equipped with a nitrogen inlet, condenser and thermometer. The reactor was heated to 75°C while stirring under atmospheric nitrogen. 0.5 Parts azobisisobutyronitrile was added to the reaction vessel and it was heated under reflux for 2 hours. The reaction was cooled and 100 parts of 1.25% ammonium hydroxide was added and stirred for 30 minutes. After that the isopropyl alcohol was removed and water was added to make a stable colloid. The final polymer was neutralized to a pH of 7.0. The polymer was characterized through molecular weight (GPC), surface tension, viscosity and % solids

### Example 8 - Comparison of Star and Linear Colloids

The polymers of Examples 1-4, 6, and 7 were characterized through molecular weight (GPC), surface tension, viscosity and % solids. The molecular weight and distribution were determined by Gel Permeation Chromatography (Waters 712 WISP with RI and UV detector and 10µm PLgel column), surface tension measurements by Surface Tensiomat 21 from Fisher Scientific, viscosity measurements by Brookfield DV-II+ Viscometer @25°C, 10 rpm and % solids by vacuum oven (1 hour @ 130°C).

The results are shown in Table I, below.

**Table I**

| **Polymer** | **Mol. Wt** | **Distribution** | **Surface Tension** | **Viscosity** | **% Solids** |
|---|---|---|---|---|---|
| Example 1 | 1.1e4 | 1.75 | 35 | 248 | 33 |
| Example 2 | 1.0e4 | 3.77 | 32 | 252 | 30 |
| Example 3 | 1.04e4 | 4.35 | 37 | 259 | 15 |
| Example 4 | 1.05e4 | 1.68 | 30 | 206 | 21 |
| Example 6 | 1.37e4 | 1.68 | 38 | 232 | 31.5 |
| Example 7 | 1.45e4 | 1.62 | 41 | 305 | 29 |

### Example 9 - - Evaluation As a Latex Glove Coating

The performance of the star and linear polymers of Examples 1 to 7 were evaluated by preparing latex glove fingers. A ceramic finger mold was cleaned from contaminants, rinsed, heated to 40 to 50°C and immersed for 15 to 20 seconds into the coagulant, a 20% aqueous solution of calcium nitrate. After dipping into the coagulant, the coagulant-coated mold was partially dried. The mold with coagulant was then immersed into the natural rubber latex at room temperature for one minute. The latex-coated mold was then leached in water at room temperature to remove natural rubber proteins. The leached latex deposit was then dried and dipped into a 10% solids aqueous dispersion of polymer for up to one minute. After dipping with polymer dispersion, the latex deposit was vulcanized in the oven by heating at 90 to 130°C for 15 to 30 minutes. After vulcanization, the coated rubber article was cooled and stripped from the mold. The ceramic mold was then cleaned.

The rubber gloves were tested for slip be the grip test in which the glove, with the coated surfaces facing each other, were rubbed between the fingers under moderate pressure. The results are shown in Table II, below.

**Table II**

| **Polymer** | **Description** |
|---|---|
| Example 1 | The formed rubber glove had a smooth coating of the copolymer. The copolymer coating formed a uniform film and had very good dry and wet donning properties. |
| Example 2 | The formed rubber glove had a smooth coating of the copolymer. The copolymer coating formed a uniform film and had very good dry and wet donning properties. |
| Example 3 | The formed rubber glove had a smooth coating of the copolymer. The copolymer coating formed a uniform film and had good dry and wet donning properties. |
| Example 4 | The formed rubber glove had a smooth coating of the copolymer. The copolymer coating formed a uniform film and did not delaminate upon stretching. |
| Example 5 | The formed rubber glove has a smooth coating of the copolymer. The copolymer coating forms a uniform film and has good dry and wet donning properties. |
| Example 6 | The formed rubber glove had a smooth coating of the copolymer. The copolymer coating formed a uniform film and had good dry and wet donning properties. |
| Example 7 | The formed rubber glove had a dull surface finish. The suface of the glove exposed to polymer was tacky and had poor slip against the skin. |

### Example 10 - Alternate Glove Process

The above described process for preparing latex glove fingers was followed except the water was heated to about 50°C. This facilitated the leaching of the natural rubber proteins, resulting in gloves with reduced protein content.

## Claims

1. An article comprising a formed latex article having thereon a coating comprising at least one star polymer.

2. The article of claim 1, wherein the star polymer comprises at least two different monomers.

3. The artide of claim 2, wherein the monomers comprise at least one hydrophobic monomer and at least one hydrophilic monomer.

4. The article of claims 2 or 3, wherein the at least two different monomers have different glass transition temperatures.

5. The article of claim 1, wherein the star polymer is a heteroarm star copolymer.

6. The article of claim 1, wherein the star polymer is formed with a core selected from the group consisting of pentaerythritol tetrakis(3-mercaptopropionate); pentaerythritol tetrakis(2-mercaptoacetate); and pentaerythritol tetrakis(2-mercaptopropionate).

7. The article of claims 1-3, wherein the hydrophobic monomer is selected from the group consisting of methyl methacrylate, methyl acrylate, n-butyl methacrylate, n-butyl acrylate, 2-ethylhexyl methacrylate, 2-ethylhexyl acrylate, lauryl methacrylate, lauryl acrylate, and t-octyl acrylamide.

8. The article of claims 1-3, wherein the hydrophilic monomer is selected from the group consisting of methacrylic acid, acrylic acid, 2-acrylamido-2-methyl propane sulfonic acid and the alkali, alkaline earth or ammonium salts thereof, and [3-(methacryloylamino)propyl]trimethylammonium chloride.

9. The article of claims 1-5, wherein the star polymer has at least one arm with a T_{g} of at least about 20°C, preferably at least about 40°C, and at least one with a T_{g} of less than about 20°C, and preferably less than about 0°C.

10. The article of claims 1-5, wherein the monomers have crosslinkable functionality.

11. The article of claim 10, wherein the monomer is selected from the group consisting of hydroxyalkyl esters of ethylenically unsaturated carboxylic acids, ethylenically unsaturated carboxylic acids, ethylenically unsaturated epoxides and ethylenically unsaturated isocyanates.

12. The article of claim 10 wherein the crosslinkable monomers are selected from the group consisting of vinyltriisopropoxysilane, vinyltrimethoxysilane, vinyltriethoxysilane, vinyl-tris(2-methoxy-ethoxy)silane, and gamma-methacryloxyoropyltrimethoxysilane.

13. The artide of claims 1-5, which further comprises a low surface energy modifier.

14. The article of claims 1-5, wherein the article is selected from the group consisting of gloves, prophylactics, catheters, balloons, tubing, and sheeting.

15. The article of claim 14, wherein the article is a glove, preferably powder-free, selected from the group consisting of surgeons' gloves, physicians' examining gloves, and workers' gloves.

16. A method of making a glove comprising:
a) dipping a former into a coagulant that comprises a mold release agent, removing the former from the coagulant and drying it to form a mold-releasing layer on the former;
b) dipping the former into rubber latex and drying it to form a partially-cured rubber deposit on top of mold-release layer;
c) dipping the deposit of rubber into a dispersion of at least one star polymer, and drying it to form a lubricating layer on the rubber deposit;
d) vulcanizing the deposit of rubber with the mold-releasing and lubricating layers in an oven at about 100°C until the rubber is vulcanized to the desired degree and the layers are bonded to the rubber; and
e) cooling and then removing a finished glove from the said ceramic former.

17. The method of claim 16, wherein the mold release agent of the coagulant is at least star polymer.

18. The method of claims 16 or 17, further comprising after step (b) dipping the partially cured rubber deposit into water for sufficient time to remove at least some soluble proteins and other contaminants from the partially cured rubber deposit to form a leached partially cured rubber deposit.

## Patentansprüche

1. Gegenstand, umfassend einen geformten Latex-Gegenstand, der darauf eine Beschichtung hat, die mindestens ein Sternpolymer umfasst.

2. Gegenstand nach Anspruch 1, wobei das Sternpolymer mindestens zwei unterschiedliche Monomere umfasst.

3. Gegenstand nach Anspruch 2, wobei die Monomere mindestens ein hydrophobes Monomer und mindestens ein hydrophiles Monomer umfassen.

4. Gegenstand nach Anspruch 2 oder Anspruch 3, wobei die mindestens zwei unterschiedlichen Monomere unterschiedliche Glasübergangstemperaturen haben.

5. Gegenstand nach Anspruch 1, wobei das Sternpolymer ein Heteroarmsterncopolymer ist.

6. Gegenstand nach Anspruch 1, wobei das Sternpolymer mit einem Kern, ausgewählt aus der Gruppe, bestehend aus Pentaerythrol-tetrakis(3-mercaptopropionat), Pentaerythrol-tetrakis(2-mercaptoacetat) und Pentaerythroltetrakis(2-mercaptopropionat), geformt ist.

7. Gegenstand nach den Ansprüchen 1 bis 3, wobei das hydrophobe Monomer aus der Gruppe, bestehend aus Methylmethacrylat, Methylacrylat, N-Butylmethacrylat, n-Butylacrylat, 2-Ethylhexylmethacrylat, 2-Ethylhexylacrylat, Laurylmethacrylat, Laurylacrylat und t-Octylacrylamid, ausgewählt ist.

8. Gegenstand nach den Ansprüchen 1 bis 3, wobei das hydrophile Monomer aus der Gruppe, bestehend aus Methacrylsäure, Acrylsäure, 2-Acrylamido-2-methylpropansulfonsäure und den Alkali-, Erdalkali- und Ammoniumsalzen derselben und [3-(Methacryloylamino)propyl]trimethylammoniumchlorid, ausgewählt ist.

9. Gegenstand nach den Ansprüchen 1 bis 5, wobei das Sternpolymer mindestens einen Arm mit einer T_{g} von mindestens etwa 20°C, vorzugsweise mindestens etwa 40°C, und mindestens einen mit einer T_{g} von weniger als etwa 20°C und vorzugsweise weniger als etwa 0°C hat.

10. Gegenstand nach den Ansprüchen 1 bis 5, wobei die Monomere vernetzbare Funktionalität haben.

11. Gegenstand nach Anspruch 10, wobei das Monomer ausgewählt ist aus der Gruppe, bestehend aus Hydroxyalkylestern von ethylenisch ungesättigten Carbonsäuren, ethylenisch ungesättigten Carbonsäuren, ethylenisch ungesättigten Epoxiden und ethylenisch ungesättigten Isocyanaten.

12. Gegenstand nach Anspruch 10, wobei die vernetzbaren Monomere aus der Gruppe, bestehend aus Vinyltriisopropoxysilan, Vinyltrimethoxysilan, Vinyltriethoxysilan, Vinyl-tris(2-methoxy-ethoxy)silan und gamma-Methacryloxypropyltrimethoxysilan, ausgewählt sind.

13. Gegenstand nach den Ansprüchen 1 bis 5, der außerdem ein Modifizierungsmittel für geringe Oberflächenenergie umfasst.

14. Gegenstand nach den Ansprüchen 1 bis 5, wobei der Gegenstand aus der Gruppe, bestehend aus Handschuhen, Prophylaktika, Kathetern, Ballons, Schlauch- und Folienmaterial, ausgewählt ist.

15. Gegenstand nach Anspruch 14, wobei der Gegenstand ein Handschuh, vorzugsweise ein pulverfreier Handschuh ist, ausgewählt aus der Gruppe bestehend aus Chirurgenhandschuhen, Arztuntersuchungshandschuhen und Arbeitshandschuhen.

16. Verfahren zur Herstellung eines Handschuhs, umfassend:
a) Eintauchen einer Form in ein Koagulans, das ein Formentrennmittel umfasst, Entfernen der Form aus dem Koagulans und Trocknen derselben unter Bildung einer Formtrennmittelschicht auf der Form;
b) Eintauchen der Form in Kautschuklatex und Trocknen derselben unter Bildung einer teilweise gehärteten Kautschukabscheidung auf der Formtrennmittelschicht;
c) Eintauchen der Kautschukabscheidung in eine Dispersion aus mindestens einem Sternpolymer und Trocknen derselben unter Bildung einer Gleitmittelschicht auf der Kautschukabscheidung;
d) Vulkanisieren der Kautschukabscheidung mit den Formtrennmittelund Gleitmittelschichten in einem Ofen bei etwa 100°C, bis der Kautschuk zum gewünschten Grad vulkanisiert ist und die Schichten an den Kautschuk gebunden sind; und
e) Abkühlen und danach Entfernen des fertigen Handschuhs von der Keramikform.

17. Verfahren nach Anspruch 16, wobei das Formentrennmittel des Koagulans mindestens ein Sternpolymer ist.

18. Verfahren nach Anspruch 16 oder 17, das außerdem nach Schritt b) Eintauchen der teilweise gehärteten Kautschukabscheidung in Wasser für eine ausreichende Zeit umfasst, um mindestens einige lösliche Proteine und andere Kontaminanten von der teilweise gehärteten Kautschukabscheidung zu entfernen, um eine gelaugte, teilweise gehärtete Kautschukabscheidung zu bilden.

## Revendications

1. Article comprenant un article en latex façonné portant un revêtement comprenant au moins un polymère en étoile.

2. Article suivant la revendication 1, dans lequel le polymère en étoile comprend au moins deux monomères différents.

3. Article suivant la revendication 2, dans lequel les monomères comprennent au moins un monomère hydrophobe et au moins un monomère hydrophile.

4. Article suivant la revendication 2 ou 3, dans lequel lesdits au moins deux monomères différents possèdent des températures de transition vitreuse différentes.

5. Article suivant la revendication 1, dans lequel le polymère en étoile est un copolymère en étoile à branches hétérogènes.

6. Article suivant la revendication 1, dans lequel le polymère en étoile est formé avec un noyau choisi dans le groupe consistant en tétrakis(3-mercaptopropionate) de pentaérythritol, tétrakis(2-mercaptoacétate) de pentaérythitol et tétrakis(2-mercaptopropionate) de pentaérythritol.

7. Article suivant les revendications 1 à 3, dans lequel le monomère hydrophobe est choisi dans le groupe consistant en méthacrylate de méthyle, acrylate de méthyle, méthacrylate de n-butyle, acrylate de n-butyle, méthacrylate de 2-éthylhexyle, acrylate de 2-éthylhexyle, méthacrylate de lauryle, acrylate de lauryle et tertiooctylacrylamide.

8. Article suivant les revendications 1 à 3, dans lequel le monomère hydrophile est choisi dans le groupe consistant en acide méthacrylique, acide acrylique, acide 2-acrylamido-2-méthylpropanesulfonique et leurs sels de métaux alcalins, de métaux alcalinoterreux ou d'ammonium et chlorure de [3-(méthacryloylamino)propyl]triméthylammonium.

9. Article suivant les revendications 1 à 5, dans lequel le polymère en étoile possède au moins une branche avec une T_{g} d'au moins 20°C, de préférence d'au moins environ 40°C, et au moins une branche avec une T_{g} inférieure à environ 20°C et de préférence inférieure à environ 0°C.

10. Article suivant les revendications 1 à 5, dans lequel les monomères ont une fonctionnalité réticulable.

11. Article suivant la revendication 10, dans lequel le monomère est choisi dans le groupe consistant en des esters hydroxyalkyliques d'acides carboxyliques à insaturation éthylénique, des acides carboxyliques à insaturation éthylénique, des époxides à insaturation éthylénique et des isocyanates à insaturation éthylénique.

12. Article suivant la revendication 10, dans lequel les monomères réticulables sont choisis dans le groupe consistant en vinyltriisopropoxysilane, vinyltriméthoxysilane, vinyltriéthoxysilane, vinyltris(2-méthoxyéthoxy)silane, et gammaméthacryloxyoropyltriméthoxysilane.

13. Article suivant les revendications 1 à 5, qui comprend en outre un modificateur de faible énergie superficielle.

14. Article suivant les revendications 1 à 5, qui est choisi dans le groupe consistant en des gants, des articles phophylactiques, des cathéters, des ballonnets, des tubulures et des feuilles.

15. Article suivant la revendication 14, qui est un gant, de préférence dépourvu de poudre, choisi dans le groupe consistant en des gants de chirurgie, des gants d'examens médicaux et des gants de travailleurs.

16. Procédé pour la production d'un gant, comprenant les étapes consistant :
a) à plonger une forme dans un coagulant qui comprend un agent de démoulage, à enlever la forme du coagulant et à la sécher pour former une couche d'agent de démoulage sur la forme ;
b) à plonger la forme dans un latex de caoutchouc et à la sécher pour former un dépôt de caoutchouc partiellement durci sur la couche d'agent de démoulage ;
c) à plonger le dépôt de caoutchouc dans une dispersion d'au moins un polymère en étoile, et à le sécher pour former une couche lubrifiante sur le dépôt de caoutchouc ;
d) à vulcaniser le dépôt de caoutchouc avec la couche d'agent de démoulage et la couche de lubrifiant dans un four à environ 100°C jusqu'à ce que le caoutchouc soit vulcanisé au degré désiré et que les couches soient liées au caoutchouc ; et
e) à refroidir et ensuite enlever le gant fini de ladite forme céramique.

17. Procédé suivant la revendication 16, dans lequel l'agent de démoulage du coagulant est au moins un polymère en étoile.

18. Procédé suivant la revendication 16 ou 17, comprenant en outre, après l'étape (b), l'opération consistant à plonger le dépôt de caoutchouc partiellement durci dans de l'eau pendant un temps suffisant pour éliminer au moins certaines protéines solubles et d'autres contaminants du dépôt de caoutchouc partiellement durci afin de former un dépôt de caoutchouc partiellement durci ayant subi un lessivage.
